# EUROPEAN PATENT SPECIFICATION

(11) **EP 4 465 042 B1**
(45) Date of publication and mention of the grant of the patent: **28.10.2026**
(21) Application number: 24204188.7
(22) Date of filing: 14.08.2019
(51) Int. Cl.: G01N 33/493, G01N 33/50

(54) **METHOD FOR EXAMINING CANCER IN PET ANIMAL**
VERFAHREN ZUR UNTERSUCHUNG VON KREBS BEI HAUSTIEREN
PROCÉDÉ D'EXAMEN DU CANCER CHEZ UN ANIMAL DE COMPAGNIE

(30) Priority: 14.08.2018 JP 2018152607
(43) Date of publication of application: 20.11.2024
(62) Document number(s) of the earlier application(s) in accordance with Art. 76 EPC: 19849382.7 / 3 839 506
(73) Proprietor: Hirotsu Bio Science Inc., Tokyo 102-0094 (JP)
(72) Inventor: HIROTSU, Takaaki, 102-0094 Tokyo (JP); KAIFUCHI, Satoru, 102-0094 Tokyo (JP); SUGIMOTO, Toshimi, 102-0094 Tokyo (JP); ISHII, Hideshi, 565-0871 Osaka (JP); KONNO, Masamitsu, 565-0871 Osaka (JP); UEDA, Yuji, 565-0871 Osaka (JP); KOBAYASHI, Shogo, 565-0871 Osaka (JP); EGUCHI, Hidetoshi, 565-0871 Osaka (JP); DOKI, Yuichiro, 565-0871 Osaka (JP); MORI, Masaki, 565-0871 Osaka (JP)
(74) Representative: Hoffmann Eitle

(56) References cited:
- WO-A1-2020/218501
- TAKAAKI HIROTSU ET AL: "A Highly Accurate Inclusive Cancer Screening Test Using Caenorhabditis elegans Scent Detection", PLOS ONE, vol. 10, no. 3, 3 November 2015 (2015-11-03), pages e0118699, XP055311189, DOI: 10.1371/journal.pone.0118699
- UEDA YUJI ET AL: "Application of C. elegans cancer screening test for the detection of pancreatic tumor in genetically engineered mice INTRODUCTION", 1 January 2019 (2019-01-01), pages 5412 - 5418, XP055895824, Retrieved from the Internet <URL:https://www.ncbi.nlm.nih.gov/pmc/articles/PMC6739214/pdf/oncotarget-10-5412.pdf> [retrieved on 20220228]

## Description

### Technical Field

The present invention relates to a method of examining or diagnosing for cancer in a pet animal.

### Background Art

Various methods have been developed to detect cancer easily and inexpensively. Cancer in humans can be diagnosed by using the taxic behavior of nematodes as an index, that is, using the principle that nematodes show attractive behavior toward urines from human cancer patients and nematodes show avoidance behavior against urines from healthy subjects (refer to Patent Literature 1 and Non-Patent Literature 1).

### Citation List

### Patent Literature

Patent Literature 1: WO2015/088039
Non-Patent Literature 1: TAKAAKI HIROTSU ET AL: "A Highly Accurate Inclusive Cancer Screening Test Using Caenorhabditis elegans Scent Detection", PLOS ONE, vol. 10, no. 3, 3 November 2015, pages 1-15

### Summary of Invention

The present invention relates to a method of examining or diagnosing for cancer in a pet animal. The pet animal is a non-primate mammal.

The present inventors have shown that nematodes exhibit taxic behavior toward urines from pet animals having cancer, such as dogs, cats, and mice. The present invention has been based on this finding.

More specifically, the present invention is as described in the appended set of claims.

### Brief Description of Drawings

[Figure 1] Figure 1 shows the taxic behavior of nematodes toward urine from rodents (mouse) with cancer or rodents (mouse) without cancer (healthy). One bar graph corresponds to each individual.
[Figure 2] Figure 2 shows the taxic behavior of nematodes toward urines from dogs with cancer or dogs without cancer (healthy). One bar graph corresponds to each individual.
[Figure 3] Figure 3 shows the taxic behavior of nematodes toward urines from cats with cancer or cats without cancer (healthy). One bar graph corresponds to each individual.

### Detailed Description of the Invention

In the present description, the term "subject" means a pet animal. In the present description, the term "subject" is also used to mean a healthy subject, a subject suspected of having cancer, and a subject suffering from cancer. In the present description, the term "subject suspected of having cancer" includes not only a subject that is clearly suspected of having cancer but also a subject that has not been specifically suspected of having cancer.

In the present description, the term "cancer" means a malignant tumor. Cancers can be broadly classified into hematopoietic tumors, epithelial cancers (carcinoma), and non-epithelial sarcomas (sarcomas). Examples of the hematopoietic tumors include leukemia, malignant lymphoma, and myeloma. Examples of the epithelial cancers include lung cancer, breast cancer, stomach cancer, colon cancer, liver cancer, uterine cancer, ovarian cancer, head and neck cancer, and tongue cancer. Examples of the sarcomas include osteosarcomas, chondrosarcomas, rhabdomyosarcomas, leiomyosarcoma, fibrosarcomas, liposarcomas, and angiosarcomas.

In the present description, the term "detecting cancer" can be read as "detecting cancer cells", "identifying cancer", "judging cancer", "method for assisting in cancer diagnosis", or "method for obtaining preliminary information for cancer diagnosis". The method of the present invention is an industrially applicable method.

In the present description, the term "nematode" means *Caenorhabditis elegans.* For the nematode, strains isolated from various environments are registered and published at the Caenorhabditis Genetics Center (CGC) of the Faculty of Biological Sciences, University of Minnesota, USA, and can be distributed. Therefore, those skilled in the art can obtain most of known strains from CGC. Hermaphrodite can be preferably used from the viewpoint of being able to reproduce by selffertilization.

In the present description, the term "taxic behavior" means attractive behavior or avoidance behavior. The attractive behavior means the behavior of decreasing the physical distance from a certain substance, and the avoidance behavior means the behavior of increasing the physical distance from a certain substance. A substance that induces attractive behavior is called an attractant, and a substance that induces avoidance behavior is called a repellent. Nematodes have the property of being attracted to attractants and repelling repellents by the sense of smell. The behavior of being attracted toward attractants is called attractive behavior (sometimes referred to as "positive" in the present description), and the behavior of repelling repellents is referred to as avoidance behavior (sometimes referred to as "negative" in the present description). In addition, the attractive behavior and the avoidance behavior are collectively called taxic behavior.

In the present description, the term "wild strain" is a wild strain of nematodes, and an example thereof includes N2 Bristol strain, which is a general wild strain.

The present invention provides a method for detecting cancer in a subject that has or is suspected of having cancer (or a method of prediction, a method of diagnosis, or a method for obtaining basic information on the therapeutic effect), comprising evaluating the taxic behavior of nematodes toward a urine sample from the subject.

The subject is a pet animal, preferably a rodent selected from mouse, rat, hamster, chinchilla, rabbit, guinea pig, mouse, prairie dog, momonga, and squirrel, a canine (for example, dog, fox, raccoon dog, and fennec fox), and a feline (for example, cat, domestic cat, and wildcat), which can be used as the subject in the present invention. The pet animal is a non-primate mammal.

Wild-type nematodes (for example, N2 Bristol strain) can be used as the nematodes. The nematode strain exhibits attractive behavior toward a urine from a cancer patient, and therefore such nematodes exhibiting the attractive behavior toward a urine from a cancer patient can be used as the nematodes in the present invention.

A urine sample is obtained from a subject. The urine sample is diluted with a solvent such as water when evaluated by the method of the present invention. The dilution factor is 5-fold to 20000-fold, preferably the dilution factor is 10-fold to 10000-fold, more preferably the dilution factor is 10-fold to 5000-fold, even more preferably the dilution factor is 10-fold to 2000-fold, and even more preferably the dilution factor is 100-fold to 1000-fold, and the nematode also exhibits taxic behavior toward diluted urine samples. In some aspects of the present invention, the dilution factor of urine can be, for example, 100-fold or 1000-fold, or the dilution factors can be in combination of two or more, including 100-fold and 1000-fold. For example, the dilution factor of the dog urine sample may be 10-fold to 2000-fold, for example, 100-fold to 1000-fold. For example, the dilution factor of the cat may be 5-fold to 20000-fold, and the dilution factor may be 10-fold to 10000-fold, for example, 500-fold to 5000-fold.

The taxic behavior of nematodes is evaluated by placing a urine sample and nematodes at respective positions that are separated at a certain distance (for example, a distance of about 1 cm to about 5 cm) and then observing whether the nematodes exhibit attractive behavior toward or avoidance behavior against the urine sample. The taxic behavior can be observed on a solid medium such as an agar medium.

### Method of analyzing urine with nematodes

The analysis of urine with nematodes is performed by placing the test sample (i.e., urine) obtained from the subject and the nematodes at respective positions that are separated at a certain distance, and then by observing whether the nematodes exhibit attractive behavior toward or avoidance behavior against the test sample. When the nematodes exhibit attractive behavior, the subject can be evaluated to suffer from cancer or to have the possibility of having cancer. When the nematodes exhibit avoidance behavior, the subject can be evaluated to have no cancer or to have the possibility of having no cancer. An example of the analysis method with a human specimen is as disclosed in WO2015/088039.

More specifically, examples of the method of analyzing urine with nematodes include:
placing the test sample (for example, urine sample) obtained from the subject onto a petri dish (for example, a petri dish into which a solid medium has been introduced);
placing the nematodes separated at a certain distance from the test sample in the petri dish where the test sample has been placed; and then
allowing the nematodes to move.

The method of analyzing urine with nematodes can include determining that the subject suffers from cancer or has the possibility of suffering from cancer when the nematodes exhibit taxic behavior toward the test sample.

The taxic behavior of nematodes is evaluated by a difference or ratio between the number of nematodes approaching the test sample and the number of nematodes far from the test sample. In evaluation based on a difference in the numbers of nematodes, a plus value difference allows the test sample to be evaluated overall as inducing attractive behavior and being derived from a cancer patient, and/or a minus value difference allows the test sample to be evaluated overall as inducing avoidance behavior and being derived from a subject without cancer. In evaluation based on the ratio of the numbers of nematodes, a ratio of 1 or more allows the test sample to be evaluated as inducing attractive behavior and being derived from a subject having cancer, and/or a ratio of less than 1 allows the test sample to be evaluated as inducing avoidance behavior and being derived from a subject without cancer. In addition, the taxic behavior of nematodes can be evaluated by using the taxis index as an index, for example, as follows. [Formula 1] $Taxis Index = \frac{A - B}{A + B}$In the formula, A is the number of nematodes that exhibit attractive behavior toward the test sample, and B is the number of nematodes that exhibit avoidance behavior against the test sample.

A plus value of taxis index allows the test sample to be evaluated overall as inducing attractive behavior and being derived from the subject with cancer, and a minus value of taxis index allows the test sample to be evaluated as inducing avoidance behavior and being derived from the subject without cancer.

The taxis index closer to 1 means a larger proportion of nematodes that exhibit attractive behavior, the taxis index closer to -1 means a larger proportion of nematodes that exhibit avoidance behavior, and the taxis index closer to 0 means that nematodes exhibit neither attractive behavior nor avoidance behavior. A larger absolute value of the taxis index gives a clearer evaluation result of the behavior. If the nematodes that exhibit taxic behavior and the nematodes that exhibit avoidance behavior are similar in the number, it may be then evaluated as positive (in the case of positive, more detailed examination may be performed) or may be excluded from evaluation. In the present invention, an individual who is the source of a positive urine sample can be subjected to a further detailed examination to determine whether or not the individual has cancer.

In the case of examining urine samples with two or more of different dilution factors, any one of the urine samples is positive, which may be determined as cancer from the viewpoint of improving sensitivity, or all of the urine samples are positive, which may be determined as cancer from the viewpoint of improving specificity.

The subjects that have been evaluated to have cancer by the method of the present invention can then be treated for cancer (for example, chemotherapy, radiation therapy, surgical resection, and a combination thereof). The subjects that have been evaluated to have cancer by the method of the present invention may undergo more detailed examination to receive a definitive diagnosis of cancer.

### Example

### Example 1: Cancer test with rodent urine

In the present example, the taxic behavior of nematodes toward rodent urine samples was evaluated.

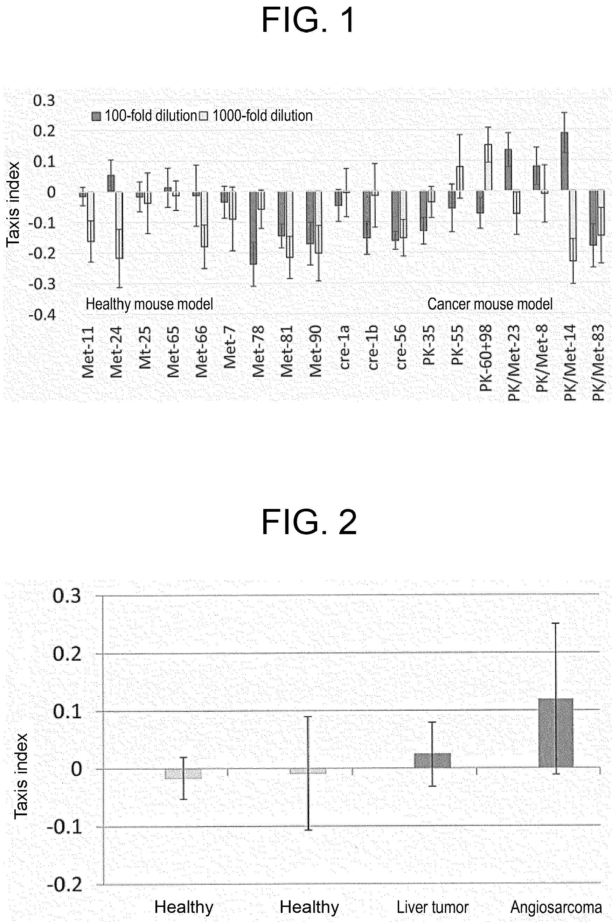

Mice were used as a rodent. Urine samples were collected from healthy mice and mice having cancer. Mice having c-Met deficiency and developing no cancer (C57Bl/6 strain) were used as healthy mice, and mice having KrasG12D mutation (refer to Noguchi K. et al., Oncology Letters, 16: 1892-1898, 2018) were used as mice having cancer. The urine samples were diluted 100-fold and the taxic behavior of nematodes toward the urine samples were evaluated by the method described in WO2015/088039. Wild-type nematodes (N2 Bristol strain) were used as the nematodes. The taxic behavior was evaluated by using the taxis index. Measurements were repeated 12 times (the same applies hereinafter) to obtain the average value of the taxis index for each individual. The results of taxic behavior toward the urine samples were as shown in Figure 1. [Formula 1] $\left(Taxis Index\right) = \frac{A - B}{A + B}$ In the formula, A is the number of nematodes that exhibit attractive behavior toward the test sample, and B is the number of nematodes that exhibit avoidance behavior against the test sample.

As shown in Figure 1, the taxis index of urine samples from healthy mice was a minus value or 0.05 or less for almost all cases, whereas the taxis index of urines from mice with cancer was more than 0.05 for urines from three strains. This revealed that mice with cancer can be evaluated by evaluating the taxic behavior of nematodes toward urine samples. When the PK - 60 + 98 urine sample was used at a 1000-fold dilution, the nematodes exhibited attractive behavior toward the urine sample (the taxis index was about 0.15). In the case of 1000-fold dilution, the nematodes exhibited attractive behavior toward urine samples from mice with three cancers. As described above, it was revealed that the nematodes exhibited attractive behavior toward urines from the cancer mouse model in both the 100-fold dilution and the 1000-fold dilution. The plus taxis index was evaluated as positive and the minus taxis index was evaluated as negative, and as a result of such evaluation, 100-fold dilution showed a sensitivity of 28.6% and a specificity of 100%, and 1000-fold dilution showed a sensitivity of 42.8% and a specificity of 83.3%. It was thus revealed that the presence or absence of cancer can be evaluated by evaluating the taxic behavior of nematodes toward rodent urine.

In addition, the plus taxis index is evaluated as positive in either the 100-fold dilution or the 1000-fold dilution, and the minus taxis index is evaluated as negative in both the 100-fold dilution and the 1000-fold dilution, and as a result of such evaluation, the sensitivity is 71.4% (five mice out of seven mice) and the specificity was 83.3% (10 mice out of 12 mice). This result revealed that the examination for cancer with the taxic behavior of nematodes as an index is effective for rodent mice.

Thereafter, urine samples were collected from healthy dogs and dogs with cancer, respectively, and the taxic behavior of nematodes was evaluated by using urine diluted 1000-fold in the same manner as in Example 1.

Healthy Chihuahuas, healthy Jack Russell Terriers, golden retrievers with hemangiosarcoma, and Scottish terriers with liver tumors were used as dogs. The results were as shown in Figure 2. As shown in Figure 2, the taxis index for urines from healthy dogs was minus, whereas the taxis index for urines from dogs with cancer was plus. In dogs, both sensitivity and specificity were 100%. This result revealed that the examination for cancer with the taxic behavior of nematodes as an index is effective for dogs.

Moreover, urine samples were collected from healthy cats and cats with cancer, respectively, and the taxic behavior of nematodes was evaluated by using urine diluted 1000-fold.

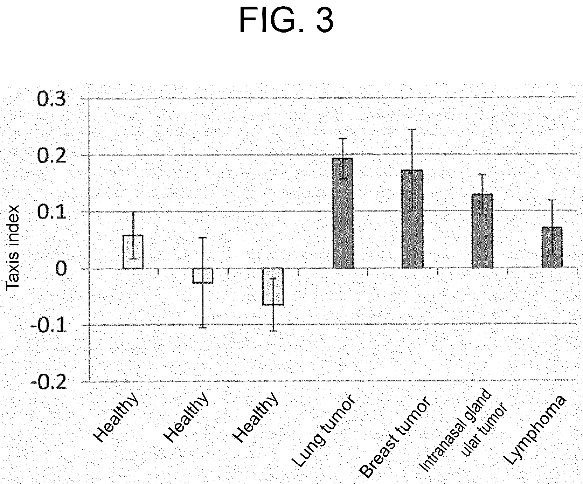

Healthy exotic shorthair (two cats), healthy Mix, American shorthair with lung tumor, Mix with breast tumor, Mix with intranasal adenocarcinoma, and Persian with lymphoma were used as cats. The results were as shown in Figure 3. As shown in Figure 3, the taxis index for urines from healthy cats was clearly lower than the taxis index for urines from cats with cancer. The plus taxis index was evaluated as positive and the minus taxis index was evaluated as negative, and as a result of such evaluation, the sensitivity was 100% (four cats out of four cats) and the specificity was 66.7% (two cats out of three cats). This result revealed that the examination for cancer with the taxic behavior of nematodes as an index is effective for cats.

The above results revealed that the examination for cancer with the taxic behavior of nematodes as an index is effective for dogs, cats, and rodent mice. As described above, pet animals can be diagnosed for cancer inexpensively and quickly by urinalysis using the taxic behavior of nematodes as an index.

In the above urine samples from dogs and cats, the taxic behavior of nematodes was similarly detected by changing the dilution factor of urine. As for dogs, the urine samples derived from the dogs having liver tumors or angiosarcomas showed plus taxis indexes at least for 10-fold dilution to 1000-fold dilution. In addition, as for cats, the urine samples derived from the cats having tumors showed plus taxis indexes at least for 10-fold dilution to 10000-fold dilution.

## Claims

1. A method of examining or diagnosing for cancer in a pet animal, wherein the pet animal is a non-primate mammal, comprising:
diluting a urine sample from the pet animal by 5-fold to 20,000-fold,
placing the diluted urine sample and nematodes at respective positions that are separated at a certain distance,
allowing the nematodes to move, and
observing whether the nematodes exhibit attractive behavior toward or avoidance behavior against the diluted urine sample, wherein the taxic behavior of nematodes is evaluated by a difference or ratio between the number of nematodes approaching the urine sample and the number of nematodes far from the urine sample.

2. The method according to claim 1, wherein the pet animal is selected from a dog and a cat.

3. The method according to claim 1 or 2, wherein the pet animal is a dog.

4. The method according to claim 1 or 2, wherein the pet animal is a cat.

5. The method according to claim 1, wherein the pet animal is a rodent, wherein the rodent is mouse, rat, hamster, chinchilla, rabbit, guinea pig, mouse, prairie dog, momonga, or squirrel.

## Patentansprüche

1. Verfahren zur Untersuchung oder Diagnose von Krebs bei einem Haustier, wobei das Haustier ein Nichtprimaten-Säugetier ist, umfassend
das 5- bis 20.000-fache Verdünnen einer Urinprobe des Haustiers,
Anordnen der verdünnten Urinprobe und Nematoden an jeweiligen Positionen, die durch einen bestimmten Abstand voneinander getrennt sind,
Erlauben der Nematoden sich zu bewegen und
Beobachten, ob die Nematoden gegenüber der verdünnten Urinprobe ein Anziehungs- oder ein Vermeidungsverhalten zeigen, wobei das Taxiverhalten der Nematoden anhand einer Differenz oder eines Verhältnisses zwischen der Anzahl der sich der Urinprobe nähernden Nematoden und der Anzahl der von der Urinprobe entfernten Nematoden bewertet wird.

2. Verfahren gemäß Anspruch 1, wobei das Haustier ausgewählt ist aus einem Hund und einer Katze.

3. Verfahren gemäß Anspruch 1 oder 2, wobei das Haustier ein Hund ist.

4. Verfahren gemäß Anspruch 1 oder 2, wobei das Haustier eine Katze ist.

5. Verfahren gemäß Anspruch 1, wobei das Haustier ein Nagetier ist, wobei das Nagetier eine Maus, eine Ratte, ein Hamster, ein Chinchilla, ein Kaninchen, ein Meerschweinchen, eine Maus, ein Präriehund, ein Momonga oder ein Eichhörnchen ist.

## Revendications

1. Procédé d'examen ou de diagnostic du cancer chez un animal de compagnie, dans lequel l'animal de compagnie est un mammifère non primate, comprenant
une dilution d'un échantillon d'urine provenant de l'animal de compagnie de 5 fois à 20 000 fois,
un placement de l'échantillon d'urine dilué et de nématodes à des positions respectives qui sont séparées d'une certaine distance,
le fait de permettre aux nématodes de se déplacer, et
le fait d'observer si les nématodes présentent un comportement attractif envers ou un comportement d'évitement à l'encontre de l'échantillon d'urine dilué, dans lequel le comportement taxique de nématodes est évalué par une différence ou un rapport entre le nombre de nématodes approchant l'échantillon d'urine et le nombre de nématodes éloignés de l'échantillon d'urine.

2. Procédé selon la revendication 1, dans lequel l'animal de compagnie est sélectionné parmi un chien et un chat.

3. Procédé selon la revendication 1 ou la revendication 2, dans lequel l'animal de compagnie est un chien.

4. Procédé selon la revendication 1 ou la revendication 2, dans lequel l'animal de compagnie est un chat.

5. Procédé selon la revendication 1,
dans lequel l'animal de compagnie est un rongeur,
dans lequel le rongeur est une souris, un rat, un hamster, un chinchilla, un lapin, un cochon d'Inde, une souris, un chien de prairie, un momonga ou un écureuil.
